# EUROPEAN PATENT APPLICATION

(11) **EP 4 835 476 A1**
(43) Date of publication of application: **04.11.2026**
(21) Application number: 24913113.7
(22) Date of filing: 27.12.2024
(51) Int. Cl.: G01N 33/545, C12M 1/00, C12M 1/34, C12N 15/09, G01N 37/00

(54) **EXAMINATION DEVICE INCLUDING REACTION UNIT COATED WITH REACTIVE SUBSTANCE**

(30) Priority: 28.12.2023 JP 2023222516
(71) Applicant: ZACROS Corporation, Tokyo 112-0002 (JP)
(72) Inventor: SAKAI, Kazuo, Tokyo 112-0002 (JP); HOSOKAWA, Kazuya, Tokyo 112-0002 (JP)
(74) Representative: Watermeyer, Nicholas
(86) International application number: PCT/JP2024/046415
(87) International publication number: WO 2025/143227

(57) **Abstract**

The present invention provides an inspection device comprising a substrate having a reaction section on the surface of which a reactive substance is applied, wherein the reactive substance is applied to the substrate surface at the reaction section in a state of being embedded in a hydrophilic compound having low volatility at room temperature, and the amount of the hydrophilic compound in the reaction section is 0.1 to 10.0 µL equivalent per cm². This enables the reactive substance to be stably maintained when not in use, while allowing the reactive substance to be rapidly and uniformly dispersed during the reaction, thereby improving the accuracy of measurement results obtained using the inspection device.

## Description

### Technical Field

The present invention relates to an inspection device such as a microchip used for analyzing or testing components in biological samples or environmental samples, and a method for manufacturing the same, and more particularly to an inspection device such as a microchip comprising, on a surface thereof, a reaction section coated with a reactive substance, and a method for manufacturing the same.

### Background Art

As an example of a microchip used for analyzing or testing components in biological samples or environmental samples, a microchip is known that has a reaction section containing a reactive substance such as an antibody or nucleic acid that binds to a target substance in a sample, into which the sample is introduced so that the reaction between the target substance in the sample and the reactive substance is detected and analyzed.

For example, Patent Document 1 discloses a microchip for nucleic acid amplification reaction in which a plurality of reagents necessary for the reaction are laminated and immobilized in a predetermined order within a well serving as the reaction site for the nucleic acid amplification reaction.

### Prior Art Document

### Patent Document

[Patent Document 1] Japanese Unexamined Patent Application Publication No. 2011-160728

### Summary of the Invention

### Problem to be Solved by the Invention

As described above, microchips in which a reactive substance is immobilized on a substrate to form a reaction section are known; however, when a liquid sample is added to the reaction section to carry out a reaction, problems such as non-uniform reactions caused by poor dispersibility of the reactive substance have been encountered, resulting in variation in measurement results.

An object of the present invention is to provide an inspection device comprising, on a surface thereof, a reaction section coated with a reactive substance such as a protein, sugar chain, or nucleic acid, which stably retains the reactive substance when not in use, while rapidly and uniformly dispersing the reactive substance during a reaction to improve the accuracy of measurement results.

### Means for Solving the Problem

The present inventors conducted extensive studies to solve the above problem. As a result, in fabricating an inspection device having, on a surface thereof, a reaction section coated with a reactive substance, they found that by embedding the reactive substance in a hydrophilic compound having low volatility at room temperature, such as glycerol, and applying it in a state containing a certain amount of moisture in the region of the substrate surface that serves as the reaction section, the reactive substance can be stably maintained when not in use (during storage), while the reactive substance is efficiently dispersed when a liquid is added or introduced to carry out a reaction, enabling uniform reaction and improving the accuracy of measurement results. Thus, the present invention has been accomplished.

One aspect of the present invention relates to an inspection device comprising a substrate having, on a surface thereof, a reaction section coated with a reactive substance, wherein the reactive substance has been applied to the substrate surface at the reaction section in a state of being embedded in a hydrophilic compound having low volatility at room temperature, the amount of the hydrophilic compound in the reaction section is equivalent to 0.1 to 10.0 µL per cm², and the water content of the hydrophilic compound is 5 to 80% by mass.

Another aspect of the present invention relates to a method for manufacturing an inspection device having, on a surface thereof, a reaction section coated with a reactive substance, said reactive substance being embedded in a hydrophilic compound having low volatility at room temperature, and the amount of the hydrophilic compound in the reaction section being equivalent to 0.1 to 10.0 µL per cm², the method comprising:
a step of preparing a substrate having a region to serve as a reaction section;
a step of preparing a solution or dispersion in which a reactive substance is dissolved or dispersed in an aqueous solution of a hydrophilic compound having low volatility at room temperature;
a step of adding the solution or dispersion of the reactive substance to the region of the substrate that is to serve as the reaction section; and
a step of placing the substrate under conditions of an ambient relative humidity of 10 to 90% and adjusting the water content of the hydrophilic compound contained in the reaction section to 5 to 80% by mass through moisture equilibrium.

### Effects of the Invention

According to the inspection device of the present invention, by embedding a reactive substance in a hydrophilic compound having low volatility at room temperature and applying it to the reaction section of the inspection device in a state containing a certain amount of moisture, the reactive substance can be stably maintained while suppressing scattering and deterioration over time, and since the reactive substance is efficiently dispersed when a liquid sample is added and reacted, the reaction can be carried out uniformly, thereby improving the accuracy of measurement results. Furthermore, embedding in a low volatile hydrophilic compound also has the effect of preventing freezing of the reactive substance and protecting its activity.

### Brief Description of the Drawings

[Fig. 1] A graph showing the evaluation results of variation in dye distribution with the addition of glycerol.
[Fig. 2] A graph showing the evaluation results of the time required for dye dispersion with the addition of glycerol.
[Fig. 3] Photographs showing the state of dye dispersion upon addition of liquid without glycerol addition (left) and with glycerol addition (right).
[Fig. 4] A graph showing the evaluation results of the time required for dye dispersion when diethylene glycol (top) or polyethylene glycol (bottom) is used.
[Fig. 5] A graph showing the change over time in water content due to moisture equilibrium of each hydrophilic compound at an ambient humidity of 50%.
[Fig. 6] A graph showing the effect of glycerol and other compounds on thrombin activity after frozen storage.
[Fig. 7] A graph with the water content in glycerol on the vertical axis and the relative humidity in the environment on the horizontal axis.

### Embodiments for carrying out the Invention

The inspection device of the present invention is an inspection device comprising a substrate having, on a surface thereof, a reaction section coated with a reactive substance, wherein the reactive substance has been applied to the substrate surface at the reaction section in a state of being embedded in a hydrophilic compound having low volatility at room temperature, the amount of the hydrophilic compound in the reaction section is equivalent to 0.1 to 10.0 µL per cm², and the water content is 5 to 80% by mass.

The type of inspection device is not particularly limited as long as it comprises a substrate having, on a surface thereof, a reaction section which is coated with a reactive substance and to which a liquid sample can be added, but preferred examples include microchips and test strips.

The material of the substrate of the inspection device may be metal, glass, plastic, silicone, or the like; however, from the standpoint of detecting the reaction by luminescence, color development, or visual observation, a transparent material is preferred, and transparent plastic is more preferred. Examples include polyethylene, polypropylene, polystyrene, polymethyl methacrylate, cyclic olefin polymer, cyclic olefin copolymer, polyphenylene oxide, polyethylene terephthalate, polyethylene naphthalate, polycarbonate, polyamide, polyimide, phenolic resin, epoxy resin, polyvinylidene chloride, polyvinyl chloride, ABS resin, and poly 2-methoxyethyl acrylate (PMEA) resin.

The water contact angle of the surface of the substrate to which the solution containing the hydrophilic compound having low volatility at room temperature and the reactive substance is applied is not particularly limited, but is preferably 10 to 90°, more preferably 20 to 80°, and even more preferably 50 to 70°.

In particular, when the substrate is planar, a substrate with a water contact angle of 50 to 70° on its surface is preferably used. In this case, it is sufficient that at least the portion of the substrate surface to which the reactive substance is applied has a water contact angle of 50 to 70°.

By using a substrate having a surface with a water contact angle of 50 to 70°, the solution containing the hydrophilic compound having low volatility at room temperature and the reactive substance can be efficiently spotted, and by allowing the solution to reach moisture equilibrium under constant humidity conditions, a more uniform thin film containing the reactive substance embedded in the hydrophilic compound and retaining a certain amount of moisture can be formed more satisfactorily.

Among substrates made of the above-mentioned materials, those with a water contact angle within this range may be used, or those that have been surface-treated so that the water contact angle falls within the above range may be used. Here, preferred surface treatments include application of a reagent that changes the water contact angle of the surface, plasma treatment, excimer treatment, or corona treatment. Examples of reagents that change the water contact angle of the surface include Beam Set 1461 (Arakawa Chemical Industries, Ltd.), RX-6-AQ P-2200 (Nippon Shokubai Co., Ltd.), and HFC-ASL8 (Harima Chemicals Group Co., Ltd.).

Methods for changing the water contact angle of a portion of the substrate surface include a method of masking a part of the substrate and performing plasma treatment, excimer treatment, or corona treatment, and a method of masking a part of the substrate and applying a hydrophilizing reagent or hydrophobizing reagent. The region to be masked may be either the portion to which the solution containing the hydrophilic compound having low volatility at room temperature and the reactive substance is applied, or the portion to which such solution is not applied.

As another embodiment, an inspection device may be provided in which the portion of the substrate to which the reactive substance is applied in a state of being embedded in the hydrophilic compound having low volatility at room temperature has a water contact angle of 90° or less on its surface, and the substrate portion is surrounded by one or more selected from a portion of the substrate surface having a water contact angle exceeding 90° and an outflow prevention wall that prevents outflow of the reactive substance.

In this case, the portion that has been made hydrophilic to a water contact angle of 90° or less preferably has a water contact angle of 30° or less, and more preferably 20° or less. In particular, by setting the water contact angle to 20° or less, the applied hydrophilic compound (e.g., glycerol solution) can be applied more uniformly.

Specifically, as one example, an inspection device may be provided in which the portion of the substrate to which the reactive substance is applied in a state of being embedded in the hydrophilic compound having low volatility at room temperature has a water contact angle of 90° or less on its surface, and its outer periphery is surrounded by a substrate portion having a surface water contact angle exceeding 90°.

As another example, an inspection device may be provided in which the portion of the substrate to which the reactive substance is applied in a state of being embedded in the hydrophilic compound having low volatility at room temperature has a water contact angle of 90° or less on its surface, and its outer periphery is surrounded by a substrate portion having a surface water contact angle exceeding 90° and an outflow prevention wall that prevents outflow of the reactive substance.

Furthermore, an inspection device may be provided in which the portion of the substrate to which the reactive substance is applied in a state of being embedded in the hydrophilic compound having low volatility at room temperature is surrounded by an outflow prevention wall that prevents outflow of the reactive substance.

The inspection device of the present invention is preferably one used for analysis by adding or introducing a liquid sample to the reaction section to cause a reaction between the target substance in the liquid sample and the reactive substance.

The reactive substance may be any substance that reacts with the target component (substance to be detected) in the sample, and can be appropriately selected according to the type of target component. The reactivity of the reactive substance includes biological reactions and chemical reactions, and biological reactions also include binding reactions. The binding reactions include not only the formation of covalent bonds, but also the formation of hydrogen bonds, hydrophobic interactions, and binding between a substrate and a receptor or enzyme. Reactive substances include proteins (including peptides), saccharides, nucleic acids, and low-molecular-weight compounds. Examples include substances such as antibodies that specifically bind to a target substance, enzyme proteins such as PT reagents and blood coagulation factors that use the target substance as a substrate. When the target substance is a nucleic acid, nucleic acid probes or polymerases (nucleic acid amplification enzymes) that amplify nucleic acids may also be used. Nucleic acid amplification reagents may be PCR reagents or LAMP (Loop-mediated Isothermal Amplification) reagents.

The reactive substance may also be a drug susceptibility testing medium. Examples of drug susceptibility testing media include media containing antibiotics and the like. The reactive substance may also be a general biochemical test reagent. Examples of general biochemical test reagents include various enzymes such as AST (GOT), ALT (GPT), LDH (lactate dehydrogenase) and its isozymes, ALP (alkaline phosphatase) and its isozymes, CK (creatine kinase) and its isozymes, amylase (Amy) and its isozymes, lipase, γ-GTP (γ-glutamyl transpeptidase), and cholinesterase (ChE); electrolytes and metals such as sodium (Na), potassium (K), chloride (Cl), calcium (Ca), phosphorus (P) [inorganic phosphorus (IP)], iron (Fe), and magnesium (Mg); proteins such as total protein (TP) and serum protein fraction (PF); nitrogen-containing components such as blood urea nitrogen (BUN), creatinine (Cr), uric acid (UA), bilirubin (Bil), and ammonia; lipids such as cholesterol, HDL-cholesterol (HDL-C), LDL-cholesterol (LDL-C), and neutral fat (triglyceride) (TG); and sugar-related substances such as blood glucose (BS, GLU) and glycated hemoglobin (HbA1c).

The reactive substance is applied in a state of being embedded in a hydrophilic compound having low volatility at room temperature, to the region of the substrate having a surface water contact angle of 50 to 70° that serves as the reaction section. For example, by dissolving or dispersing the reactive substance in an aqueous solution of a hydrophilic compound having low volatility at room temperature, spotting (adding) the resulting solution or dispersion to the region of the substrate that is to serve as the reaction section, and allowing moisture equilibrium to be reached under conditions of an ambient relative humidity of 10 to 90% (preferably 10 to 50%, more preferably 10 to 30%), the reactive substance becomes embedded in the hydrophilic compound and is applied to the reaction section in a state containing a certain amount of moisture.

The certain amount of water content may be 5 to 80% by mass, preferably 5 to 40% by mass, more preferably 5 to 20% by mass, and particularly preferably 5 to 10% by mass.

The viscosity of the applied substance containing the low volatile hydrophilic compound and the reactive substance in the reaction section after application and reaching moisture equilibrium is, at 4°C to 30°C, for example, 1 to 4,000 mPa·s, preferably 40 to 4,000 mPa·s, and more preferably 100 to 4,000 mPa·s.

The ambient relative humidity refers to the relative humidity inside the room where operations and the like are performed.

The hydrophilic compound having low volatility at room temperature is preferably an aliphatic hydrocarbon compound containing hydrophilic oxygen atoms that is liquid under normal temperature and pressure, has low volatility, and has a molecular weight of 80 to 1,000, preferably 80 to 600. Examples include alcohol compounds and ether compounds, and linear compounds are preferred. The hydrophilic compound preferably does not exhibit a denaturing effect on reactive substances such as proteins, but rather exhibits a stabilizing effect on reactive substances such as proteins.

Examples of hydrophilic compounds having low volatility at room temperature include water-soluble polyethers such as polyethylene glycol; polyhydric alcohols such as ethylene glycol and glycerol; ethylene oxide adducts of polyhydric alcohols such as ethylene oxide adducts of glycerol; and partial ester compounds of polyhydric alcohols such as glycerol monoesters and glycerol diesters. More preferred are glycerol, diethylene glycol, and polyethylene glycol having a molecular weight of 100 to 600 (low-molecular-weight PEG).

Hydrophilic compounds having low volatility at room temperature, such as glycerol, diethylene glycol, and polyethylene glycol having a molecular weight of 100 to 600, barely volatilize under normal temperature and pressure and exist in a liquid state, but have a strong affinity for water and are capable of retaining moisture through moisture equilibrium corresponding to the ambient humidity. For example, when an aqueous solution containing glycerol is allowed to stand at normal temperature and pressure, the water evaporates and glycerol ultimately remains; however, moisture corresponding to the moisture equilibrium remains in the glycerol, and it is believed that this trace amount of moisture provides a stabilizing effect on the reactive substance and promotes the dispersion of the reactive substance when a liquid sample is added.

In the inspection device of the present invention, the amount of the hydrophilic compound in the reaction section on the substrate surface is equivalent to 0.1 to 10.0 µL per cm². It is preferably equivalent to 1.0 to 5.0 µL per cm², more preferably equivalent to 1.0 to 3.0 µL per cm², and even more preferably equivalent to 1.3 to 2.5 µL per cm².

The amount "equivalent to 0.1 to 10.0 µL per cm²" refers to an amount in which 100% of the hydrophilic compound is present at 0.1 to 10.0 µL per cm²; in the case of glycerol, assuming a density of 1.26 mg/µL, this corresponds to 0.126 to 12.6 mg per cm². Similarly, for glycerol, the preferred range is 1.26 to 6.3 mg/cm² (1 to 5 µL/cm²), the more preferred range is 1.26 to 3.78 mg/cm² (1 to 3 µL/cm²), and the even more preferred range is 1.638 to 3.15 mg/cm² (1.3 to 2.5 µL/cm²).

For diethylene glycol, polyethylene glycol, and other compounds, the weight can similarly be calculated from the respective densities.

By setting the amount of the hydrophilic compound within the above range, the residual water content due to moisture equilibrium in the hydrophilic compound layer embedding the reactive substance in the reaction section falls within a suitable range, making it possible to efficiently form a thin film retaining the reactive substance. This allows the reactive substance to be stably maintained during storage and transportation by preventing scattering and the like, while also providing excellent dispersibility of the reactive substance when a liquid sample is added.

In the inspection device of the present invention, the reaction section may be recessed in order to allow a liquid sample to be added or introduced.

The inspection device preferably has a flow channel (inflow-side channel) connected to the reaction section and capable of supplying a liquid sample to the reaction section. Such a flow channel can be fabricated by providing a groove on the substrate surface that connects to the recess serving as the reaction section. The inspection device may also have a flow channel (outflow-side channel) connected to the reaction section and capable of allowing the liquid sample to flow out from the reaction section.

The inspection device may also be provided with a member such as a film that covers the reaction section and the flow channel. The material of the film is preferably transparent plastic, and more preferred examples include polyethylene terephthalate (PET) resin, cyclic olefin polymer (COP) resin, cyclic olefin copolymer (COC) resin, polystyrene (PS) resin, polycarbonate (PC) resin, and polymethyl methacrylate (PMMA) resin. The substrate and the film can be bonded together using an adhesive, a pressure-sensitive adhesive, or the like.

The inspection device of the present invention can be used by adding or introducing a liquid sample to the reaction section and causing the target substance in the liquid sample to react with the reactive substance. For example, by adding a liquid sample to the reaction section, the reactive substance is dispersed within the reaction section, and the target substance in the liquid sample reacts with the reactive substance. By detecting this reaction, the target substance in the liquid sample can be detected, and by quantifying the reaction, the target substance can be quantified.

The inspection device of the present invention can be manufactured, for example, by the following steps.

The method for manufacturing the inspection device of the present invention comprises:
a step of preparing a substrate having a region to serve as a reaction section;
a step of preparing a solution or dispersion in which a reactive substance is dissolved or dispersed in an aqueous solution of a hydrophilic compound having low volatility at room temperature;
a step of adding the solution or dispersion of the reactive substance to the region of the substrate that is to serve as the reaction section; and
a step of placing the substrate under conditions where the ambient relative humidity is 10 to 90% (preferably 10 to 50%, more preferably 10 to 30%) and adjusting the water content of the hydrophilic compound contained in the reaction section to 5 to 80% by mass (preferably 5 to 40% by mass, more preferably 5 to 20% by mass, and particularly preferably 5 to 10% by mass) through moisture equilibrium.

As the substrate, the substrates described above can be used. The water contact angle of the surface of the substrate is not particularly limited, but is preferably 10 to 90°, more preferably 20 to 80°, and even more preferably 50 to 70°.

For the preparation of the solution or dispersion in which the reactive substance is dissolved or dispersed in an aqueous solution of the hydrophilic compound having low volatility at room temperature, an aqueous solution of the hydrophilic compound having low volatility at room temperature may first be prepared and the reactive substance may then be dissolved or dispersed therein, or the reactive substance may be dissolved or dispersed in water and the hydrophilic compound having low volatility at room temperature may then be dissolved therein.

The concentration of the hydrophilic compound in the solution or dispersion may be any concentration suitable for ultimately placing the hydrophilic compound in the reaction section in the predetermined amount described above (0.1 to 10.0 µL equivalent per cm², preferably 1.0 to 5.0 µL equivalent per cm², more preferably 1.0 to 3.0 µL equivalent per cm², and even more preferably 1.3 to 2.5 µL equivalent per cm²), but is preferably 0.2 to 20% by mass. Meanwhile, the concentration of the reactive substance in the solution or dispersion can be appropriately adjusted based on the amount required for the reaction.

The method of adding the solution or dispersion to the region that is to serve as the reaction section of the inspection device is not particularly limited, but it is preferred to apply it dropwise using a dispenser, inkjet device, pipette, or the like. The amount added is preferably 0.5 to 50 µL. By keeping within this range, the reactive substance can be efficiently fixed to the reaction section in a state of being embedded in the hydrophilic compound.

The step of adjusting the water content of the hydrophilic compound contained in the reaction section to 5 to 80% by mass includes, for example, a method of bringing the water content of the hydrophilic compound and the water content in the environment into moisture equilibrium under constant humidity conditions (ambient relative humidity of 10 to 90%). The method of bringing the water content of the hydrophilic compound and the water content in the environment into moisture equilibrium is not particularly limited, but it is preferred, for example, to allow the substrate to stand under conditions of constant humidity at normal temperature and pressure. In the reaction section, the hydrophilic compound retains a water content of 5 to 80% by mass through moisture equilibrium. By maintaining such a state containing a trace amount of moisture, a uniform thin film containing the reactive substance can be formed, and the reactive substance can be uniformly dispersed when a liquid sample is added.

The step of adjusting the water content of the hydrophilic compound contained in the reaction section to 5 to 80% by mass can also be carried out with the inspection device sealed in a moisture-proof packaging material. For example, a method may be employed in which the inspection device is sealed in a moisture-proof packaging material and allowed to stand in an environment with controlled humidity. This allows the water content of the hydrophilic compound to reach moisture equilibrium with the water content of the gas inside the moisture-proof packaging material.

The following describes preferred embodiments of a microchip, which is one embodiment of the inspection device of the present invention. However, the inspection device of the present invention is not limited to the following embodiments.

In the inspection device of the present invention, the method of applying the reactive substance in a state of being embedded in a hydrophilic compound having low volatility at room temperature is not particularly limited, but examples include the following two methods.

The first method involves, when making a portion of a planar substrate hydrophilic, setting the water contact angle of the portions other than the portion to which the reactive substance is applied to 70° or more and the contact angle of the portion to which the reactive substance is applied to less than 50°, and then applying the reactive substance and the hydrophilic compound.

As for the combination of the water contact angle of the portions other than the portion to which the reactive substance and hydrophilic compound are applied, and the water contact angle of the portion to which the reactive substance and hydrophilic compound are applied, it is preferred that the former (portions other than the application portion) be 80° or more and the latter (the application portion) be less than 30°. It is more preferred that the former be 90° or more and the latter be less than 20°. By reducing the latter to 20° or less, the applied liquid spreads and wets the hydrophilized region. Here, hydrophilization can be carried out by surface treatments such as aqua plasma, atmospheric pressure plasma, vacuum plasma, excimer, and corona treatments, or by application of commercially available hydrophilizing reagents.

Furthermore, the reactive substance and the hydrophilic compound can be diluted in water, alcohol, a hydrophilic solvent, or the like before application. The alcohol and the hydrophilic solvent are preferably volatile compounds.

The second method involves preventing the applied liquid from flowing out beyond the application area by making the portion to which the reactive substance and the hydrophilic compound are applied (the application portion) concave (recessed) in shape or otherwise providing a physical flow barrier around the application portion.

In this case as well, it is preferred that the application portion be hydrophilized. With regard to the degree of hydrophilization, the water contact angle of the application portion is preferably 50° or less, and more preferably 20° or less. This facilitates uniform spreading and wetting of the liquid containing the reactive substance and the hydrophilic compound over the entire application portion.

In this case, the viscosity of the liquid containing the reactive substance and the hydrophilic compound is preferably 10 to 4,000 mPa·s, more preferably 20 to 200 mPa·s, and even more preferably 30 to 100 mPa·s.

A microchip, which is one embodiment of the inspection device of the present invention, comprises: a reaction section to which a reactive substance embedded in a hydrophilic compound having low volatility at room temperature is applied in a state containing a certain amount of moisture; an inflow-side flow channel connected to the reaction section for introducing liquid into the reaction section; and an outflow-side flow channel connected to the reaction section for allowing liquid to flow out from the reaction section.

The microchip may have the reaction section, the inflow-side flow channel, and the outflow-side flow channel formed inside. That is, it may be a microchip for analyzing components in a sample by passing a liquid sample through a flow channel formed inside, causing a reaction between the target substance in the liquid sample and the reactive substance applied to the reaction section in the flow channel.

In such a case, an inlet for supplying liquid to the inflow-side flow channel may be provided at the starting end of the inflow-side flow channel, and an outlet for allowing liquid to flow out to the exterior may be provided at the terminal end of the outflow-side flow channel. In another embodiment, a waste liquid reservoir for accumulating liquid may be provided at the terminal end of the outflow-side flow channel.

A microchip having the reaction section, the inflow-side flow channel, and the outflow-side flow channel formed inside as described above may be a microchip obtained by bonding a substrate and a film together. In such a case, an example is a microchip obtained by bonding, using an adhesive or the like, a substrate provided on its surface with a recess serving as a reaction section and grooves serving as flow channels (inflow-side flow channel and outflow-side flow channel), together with a film. Regarding the inlet and outlet, through-holes may be provided on the starting-end side of the inflow-side flow channel and on the terminal-end side of the outflow-side flow channel of the substrate, respectively, to serve as the inlet and outlet, or holes may be provided at the corresponding positions on the film side.

The reaction section is coated with the reactive substance embedded in a hydrophilic compound having low volatility at room temperature; the reactive substance embedded in the hydrophilic compound may be applied to the bottom surface of the recess in the substrate that serves as the reaction section.

The reaction section of the inspection device of the present invention may be partially or entirely covered or sealed by a film having moisture permeability or a film having a breathable portion. Furthermore, the exterior may be further sealed with a moisture-proof packaging material. By the reactive components inside the inspection device reaching moisture equilibrium through the humidity inside the moisture-proof packaging material, the water content and viscosity of the reactive components are maintained within a certain range.

The inspection device of the present invention may be provided as a package sealed in a moisture-proof packaging material, which is opened at the time of use. This makes it possible to stably maintain the moisture-retaining state of the reactive substance in the state of being embedded in the hydrophilic compound. Examples of the moisture-proof packaging material include aluminum pouches.

Instead of packaging in a moisture-proof packaging material, it is also possible to first bring the hydrophilic compound (glycerol) to an equilibrium state with a water content of 5 to 80% by mass in an environment of 2 to 22°C and a relative humidity of 10 to 90%, and then place the inspection device in a state where there is no passage of water vapor into or out of the device (for example, by sealing the portions of the reaction section such as the inlet and outlet of the inspection device that allow ventilation with the outside air using a moisture-proof film, while also forming the inspection device from moisture-proof members). In particular, for an inspection device (e.g., a microchip) in which a flow channel is formed by bonding a film to a flow channel molded article, a moisture-proof film is preferably used as the film.

In this case, in the state of having reached moisture equilibrium, the ratio of the volume of the flow channel of the microchip to the total volume of the reactive substance, the hydrophilic compound, and solvents or dispersion media such as water is preferably 100:1 or less (i.e., the volume of the flow channel of the microchip is preferably 100 times or less the volume of the hydrophilic compound), more preferably 50:1 or less (i.e., the volume of the flow channel of the microchip is 50 times or less the volume of the hydrophilic compound), and even more preferably 10:1 or less (i.e., the volume of the flow channel of the microchip is 10 times or less the volume of the hydrophilic compound).

When the storage environment of the inspection device becomes cold, the relative humidity of the gas inside the packaging material or flow channel increases, causing the hydrophilic compound (e.g., glycerol) to absorb surrounding moisture, thereby increasing the water content of the hydrophilic compound.

When storing the inspection device in cold regions or below-freezing environments, by setting the ratio of the volume of the microchip to the volume of the hydrophilic compound to 100:1 or less (i.e., making the volume of the flow channel of the microchip 100 times or less the volume of the hydrophilic compound), the amount of moisture absorbed by the hydrophilic compound at low temperatures is reduced, and the increase in the water content of the hydrophilic compound is limited.

The method for manufacturing the inspection device of the present invention includes, for example, a manufacturing method comprising the following steps:
a step of preparing a substrate having a region to serve as a reaction section; a step of preparing a solution or dispersion in which a reactive substance is dissolved or dispersed in an aqueous solution of a hydrophilic compound having low volatility at room temperature; a step of adding or applying the solution or dispersion of the reactive substance to the region of the substrate that is to serve as the reaction section; and a step of adjusting the water content of the hydrophilic compound contained in the reaction section to 5 to 80% by mass.

The step of preparing a substrate having a region to serve as a reaction section includes, for example, the methods described above of partially modifying the water contact angle of the substrate or providing a physical barrier.

The step of preparing a solution or dispersion in which the reactive substance is dissolved or dispersed in an aqueous solution of a hydrophilic compound having low volatility at room temperature includes, for example, mixing the reactive substance and the hydrophilic compound with a dilution medium or dispersion medium such as water so that the concentration of the hydrophilic compound becomes 10% by mass or less. The step of adding or applying the solution or dispersion of the reactive substance and the hydrophilic compound to the region of the substrate that is to serve as the reaction section includes, for example, applying 0.5 µL to 10 µL of the solution or dispersion. The step of adjusting the water content of the hydrophilic compound contained in the reaction section to 5 to 80% by mass includes, for example, drying the hydrophilic compound so that the water content becomes 5 to 80% by mass in an environment with controlled temperature and humidity, or sealing the device in a moisture-proof packaging material (e.g., having a water vapor transmission rate of 10 µg/m²/h or less) in an environment with controlled temperature and humidity (e.g., an absolute humidity of 0 to 40 g/m³) and allowing moisture equilibrium to be reached between the solution or dispersion and the gas inside the packaging material, resulting in the water content of the hydrophilic compound reaching 5 to 80% by mass.

Through the step of adjusting the water content of the hydrophilic compound contained in the reaction section to 5 to 80% by mass, moisture equilibrium is reached between the solution or dispersion and the gas inside the packaging material, and fixation of the reactive substance becomes possible in a state where the concentration of the hydrophilic compound (e.g., glycerol) is 80 to 95% by mass.

The film thickness of the solution or dispersion after reaching moisture equilibrium is preferably 500 µm or less, more preferably 200 µm or less, and even more preferably 100 µm or less.

By applying the hydrophilic compound diluted or dispersed in a dilution medium or dispersion medium and then drying or bringing it to moisture equilibrium, the film thickness of the solution or dispersion becomes thinner. A thinner film thickness results in a faster rate of redissolution of the reactive substance when the inspection device is used.

When applying the solution or dispersion, the concentration of the hydrophilic compound in the solution or dispersion is preferably 0.1 to 50% by mass or less, more preferably 0.2 to 30% by mass or less, and even more preferably 0.2 to 10% by mass or less.

The concentration of the hydrophilic compound in the solution or dispersion inside the moisture-proof packaging material is preferably 20 to 95% by mass, more preferably 80 to 95% by mass, and even more preferably 90 to 95% by mass.

The present invention will now be described in further detail by way of examples. However, the present invention is not limited to the embodiments described in the examples.

### Example 1

Evaluation was carried out using a microchip made of COP (cyclic olefin polymer) having a surface water contact angle of 70° as the substrate.

Glycerol in an amount of 0 to 2 µL was added to 12 µL of an aqueous solution containing a coloring substance, and the mixture was applied to a region of φ5 mm on the substrate surface, and moisture was removed by natural drying. After reaching moisture equilibrium, the dye distribution in the reagent-coated area was measured using an absorbance meter, and the variation in reagent distribution (coefficient of variation: CV) was determined. The water content of the glycerol after reaching moisture equilibrium by natural drying was 25% by mass.

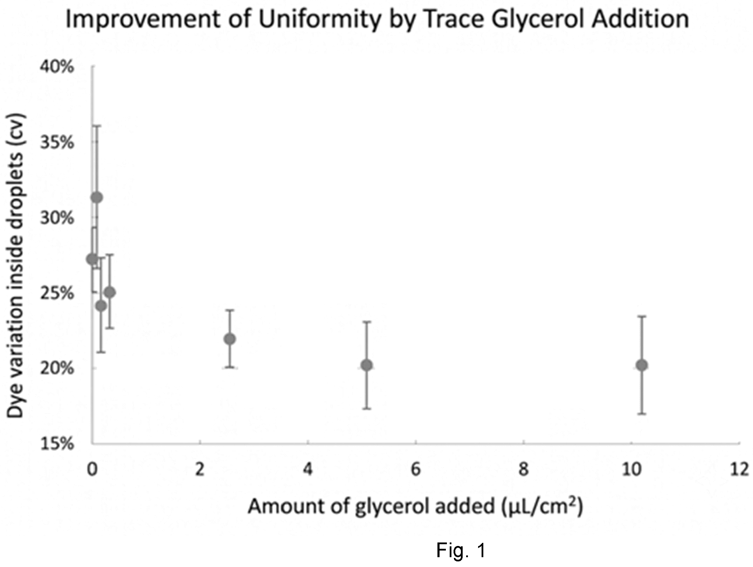

The results are shown in Fig. 1. The addition of a small amount of glycerol suppressed the variation in reagent distribution within the coated surface.

### Example 2

200 µL of physiological saline was added to the thin film formed on the substrate in Example 1, and the mixture was allowed to stand at 37°C. The time required for the applied reagent (coloring substance) to disperse throughout the entire spot was measured.

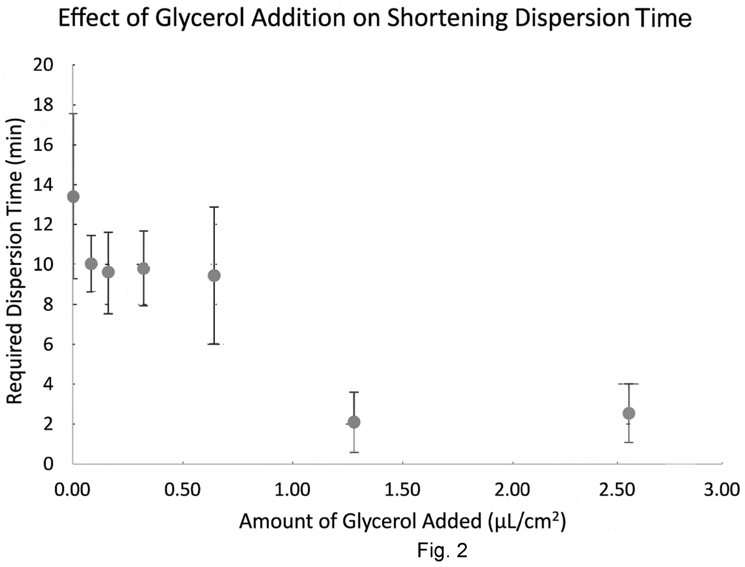

The results are shown in Fig. 2. By adding 1.3 µL or more of glycerol per square centimeter of coated area, the time required for dispersion was significantly reduced.

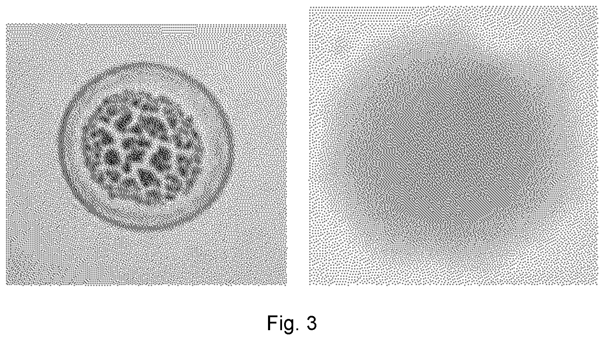

Fig. 3 shows the appearance of the coloring substance-coated surface without glycerol addition and with glycerol addition (2 µL per square centimeter of coated area) (left and right, respectively; allowed to stand and dry at 22°C for 24 hours).

Without glycerol addition, the coloring substance had precipitated and dried solid; however, with glycerol addition, the coloring substance adhered uniformly to the coated surface as a thin liquid film.

### Example 3

Substrates with surface water contact angles of 90°, 70°, and 50° were prepared, and a solution containing a coloring substance and various amounts of glycerol was applied to each substrate and allowed to air dry.

The fixation/scattering properties of the solution under these conditions were evaluated.

The results are shown in Table 1.

The fixation/scattering properties of the applied reagent depended on the contact angle between the solution and the substrate surface, as well as the amount of glycerol added. Good fixation of the glycerol-added reagent was observed in the contact angle range of 50 to 70°.

**Table 1**

| | Amount of glycerol added per cm² (µL) | | | | | | |
|---|---|---|---|---|---|---|---|
| | 0 | 0.3 | 0.6 | 1.3 | 2.5 | 5 | 10 |
| Contact angle 90° | Adhesion | Adhesion | Splashing | Splashing | Splashing | Splashing | Splashing |
| Contact angle 70° | Adhesion | Adhesion | Adhesion | Adhesion | Adhesion | Adhesion | Splashing |
| Contact angle 50° | Adhesion | Adhesion | Adhesion | Adhesion | Adhesion | Adhesion | Splashing |

### Example 4

The same experiment as in Example 2 was conducted using diethylene glycol and polyethylene glycol with a molecular weight of 200 (PEG2000) as hydrophilic compounds having low volatility at room temperature, in place of glycerol.

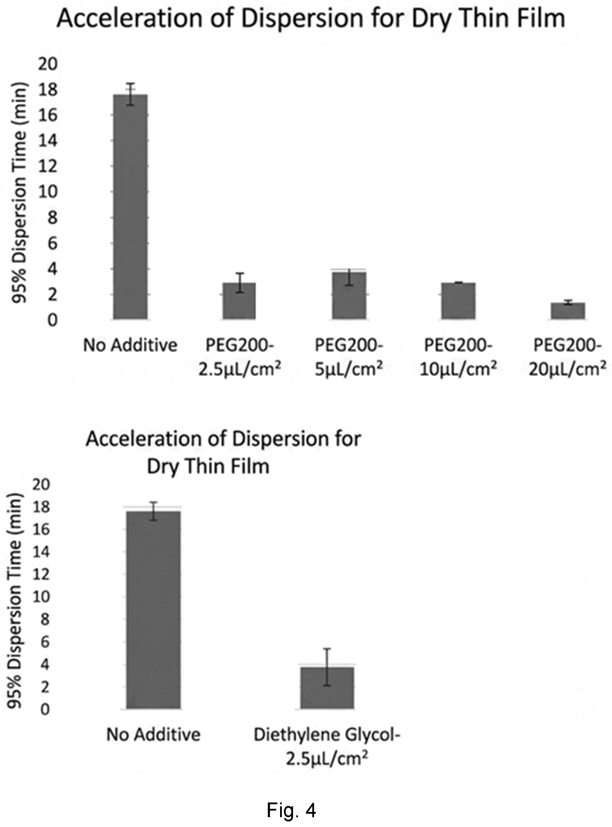

The results are shown in Fig. 4. Even when diethylene glycol and polyethylene glycol with a molecular weight of 200 (PEG200) were used, a reduction in the time required for dispersion of the coloring substance was observed.

### Example 5

The water content and moisture equilibrium of each hydrophilic compound at an ambient relative humidity of 50% were examined.

Specifically, 10 µL each of glycerol, diethylene glycol, dipropylene glycol, 3-ethoxypropane-1,2-diol, and polyethylene glycol with a molecular weight of 200 Da (PEG200) were dispensed into separate containers and allowed to stand at 22°C under a relative humidity of 50%. The mass was measured immediately after dispensing and after 13 hours, 22 hours, and 109 hours, and the water content (moisture absorption rate) was calculated using the following formula: Formula: 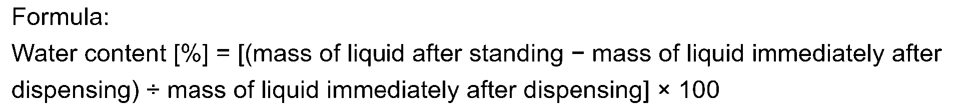 Water content [%] = [(mass of liquid after standing - mass of liquid immediately after dispensing) ÷ mass of liquid immediately after dispensing] × 100

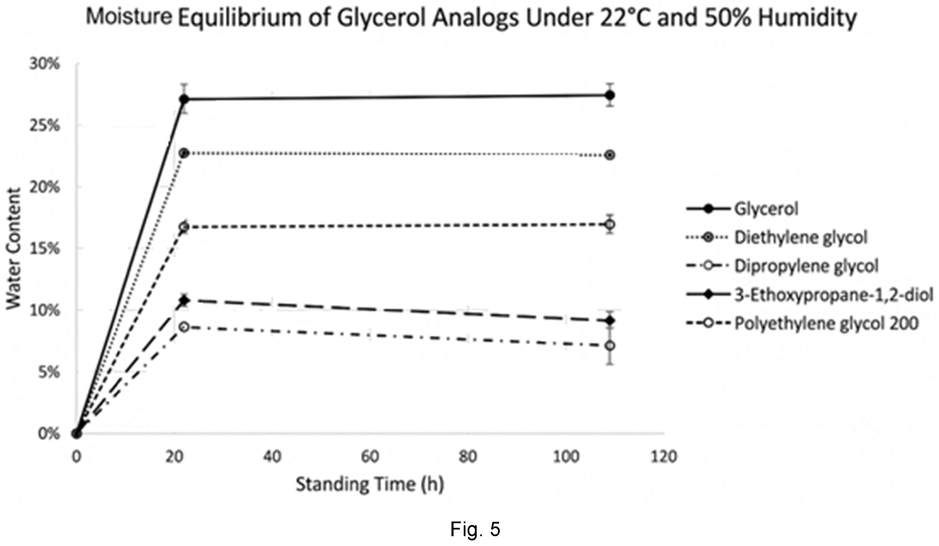

The results are shown in Fig. 5. The results demonstrated that glycerol, diethylene glycol, dipropylene glycol, 3-ethoxypropane-1,2-diol, and low-molecular-weight PEG (PEG200) all underwent rapid moisture absorption at an ambient humidity of 50% and were able to stably maintain a water content of 5 to 40% by mass over an extended period. Among these, glycerol, diethylene glycol, and low-molecular-weight PEG were able to stably maintain a water content of 15 to 30% by mass over an extended period at an ambient humidity of 50%, indicating that they are more preferred substances with less moisture volatilization and recognized long-term stability.

### Example 6

### Examination of Thrombin-Protective Effect

0.1 units of thrombin was added to a 2 mL tube manufactured by Simport, and 10 µL of a 10% by mass (1 µL/10 µL) solution of glycerol, diethylene glycol, propylene glycol, or PEG200 was added and applied thereto, and moisture equilibrium was reached at an ambient relative humidity of 50%. The sample was then stored at room temperature for 3 days, after which thrombin activity was measured. As a control, the same solution stored frozen at -30°C was used. After returning the frozen sample to room temperature, thrombin activity was measured.

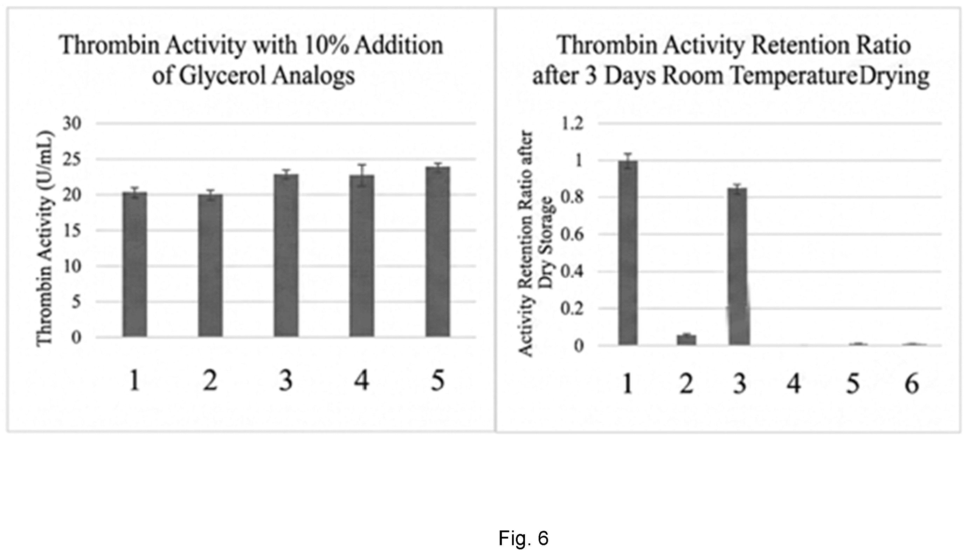

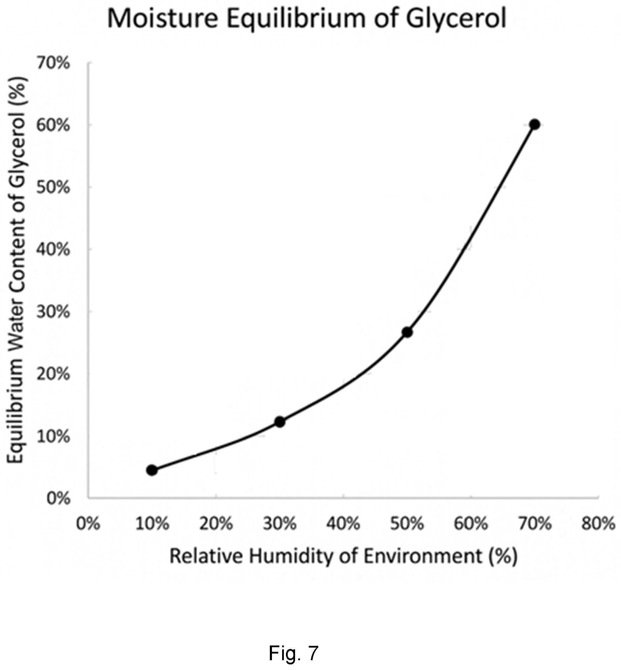

The results are shown in Fig. 6. It was found that in the absence of any additive, thrombin activity was almost completely lost after 3 days at room temperature; however, when glycerol was added, thrombin activity was retained at 80% or more even after 3 days at room temperature. Fig. 7 shows the relationship between the relative humidity in the environment and the water content in glycerol.

## Claims

1. An inspection device comprising a substrate having, on a surface thereof, a reaction section coated with a reactive substance, wherein the reactive substance has been applied to the reaction section in a state of being embedded in a hydrophilic compound having low volatility at room temperature, the amount of the hydrophilic compound in the reaction section is equivalent to 0.1 to 10.0 µL per cm², and the water content of the hydrophilic compound is 5 to 80% by mass.

2. The inspection device according to Claim 1, wherein the amount of the hydrophilic compound in the reaction section is equivalent to 1.0 to 3.0 µL per cm².

3. The inspection device according to Claim 1, wherein the hydrophilic compound is one or more selected from the group consisting of glycerol, diethylene glycol, and polyethylene glycol having a molecular weight of 100 to 600.

4. The inspection device according to Claim 1, wherein the substrate is made of one or more materials selected from the group consisting of cyclic olefin polymer, polyethylene, polypropylene, polystyrene, polymethyl methacrylate, cyclic olefin polymer, polyphenylene oxide, polyethylene terephthalate, polyethylene naphthalate, polycarbonate, polyamide, polyimide, phenolic resin, epoxy resin, polyvinylidene chloride, polyvinyl chloride, ABS resin, and poly 2-methoxyethyl acrylate (PMEA) resin.

5. The inspection device according to Claim 1, wherein the reactive substance is a protein, saccharide, or nucleic acid.

6. The inspection device according to Claim 1, wherein the reactive substance is a gene amplification reagent, a drug susceptibility test medium, or a general biochemical test reagent.

7. The inspection device according to Claim 1, which is a microchip.

8. The inspection device according to Claim 7, wherein the microchip has a flow channel connected to the reaction section for introducing liquid into the reaction section.

9. The inspection device according to Claim 8, further comprising a film bonded to the substrate so as to cover the flow channel and the reaction section of the substrate.

10. The inspection device according to Claim 1, wherein the portion of the substrate on which the reactive substance is applied in a state of being embedded in the hydrophilic compound having low volatility at room temperature has a water contact angle of 50 to 70° on its surface.

11. The inspection device according to Claim 1, wherein the portion of the substrate on which the reactive substance is applied in a state of being embedded in the hydrophilic compound having low volatility at room temperature has a water contact angle of 30 to 90° on its surface, and the substrate portion is surrounded by one or more selected from a portion having a water contact angle of the substrate surface exceeding 90° and an outflow prevention wall that prevents outflow of the reactive substance.

12. The inspection device according to any one of Claims 1 to 11, packaged in a moisture-proof packaging material.

13. A method for manufacturing an inspection device having, on a surface thereof, a reaction section coated with a reactive substance, said reactive substance being embedded in a hydrophilic compound having low volatility at room temperature, and the amount of the hydrophilic compound in the reaction section being 0.1 to 10.0 µL equivalent per cm²,
the method comprising:
a step of preparing a substrate having a region to serve as a reaction section;
a step of preparing a solution or dispersion in which a reactive substance is dissolved or dispersed in an aqueous solution of a hydrophilic compound having low volatility at room temperature;
a step of adding the solution or dispersion of the reactive substance to the region of the substrate that is to serve as the reaction section; and
a step of adjusting the water content of the hydrophilic compound contained in the reaction section to 5 to 80% by mass.

14. The method for manufacturing an inspection device according to Claim 13, wherein the amount of the solution or dispersion to be added is 10 to 50 µL.

15. The method for manufacturing an inspection device according to Claim 13, wherein the water contact angle of the portion of the substrate to which the reactive substance embedded in the hydrophilic compound having low volatility at room temperature is added is 50° to 70°.

16. The method for manufacturing an inspection device according to Claim 13, wherein the inspection device is the inspection device according to any one of Claims 1 to 12.
